(19) Europäisches Patentamt
European Patent Office
Office européen des brevets

(11) Publication number: 0 424 901 A1

# (12) EUROPEAN PATENT APPLICATION

(21) Application number: 90120381.0

(22) Date of filing: 24.10.90

(51) Int. Cl.[5]: C07D 211/90, C07D 401/12, A61K 31/445

(30) Priority: 26.10.89 JP 281138/89

(43) Date of publication of application: 02.05.91 Bulletin 91/18

(84) Designated Contracting States: BE CH DE DK ES FR GB IT LI NL SE

(71) Applicant: THE GREEN CROSS CORPORATION
3-3, Imabashi 1-chome Chuo-ku
Osaka-shi Osaka 541(JP)

(72) Inventor: Ashimori, Atsuyuki, c/o The Green Cross Corp.
Central Research Lab., 2-1180-1, Shodaiohtani
Hirakata-shi, Osaka 573(JP)
Inventor: Ohtaki, Yutaka, c/o The Green Cross Corp.
Central Research Lab., 2-1180-1, Shodaiohtani
Hirakata-shi, Osaka 573(JP)
Inventor: Fukaya, Chikara, c/o The Green Cross Corp.
Central Research Lab., 2-1180-1, Shodaiohtani
Hirakata-shi, Osaka 573(JP)
Inventor: Yokoyama, Kazumasa, c/o The Green Cross Corp.
Central Research Lab., 2-1180-1, Shodaiohtani
Hirakata-shi, Osaka 573(JP)

(74) Representative: von Kreisler, Alek, Dipl.-Chem. et al
Patentanwälte Von Kreisler-Selting-Werner, Deichmannhaus am Hauptbahnhof
W-5000 Köln 1(DE)

## (54) Dihydropyridine derivatives and pharmaceutical compositions.

(57) Dihydropyridine derivatives of the formula

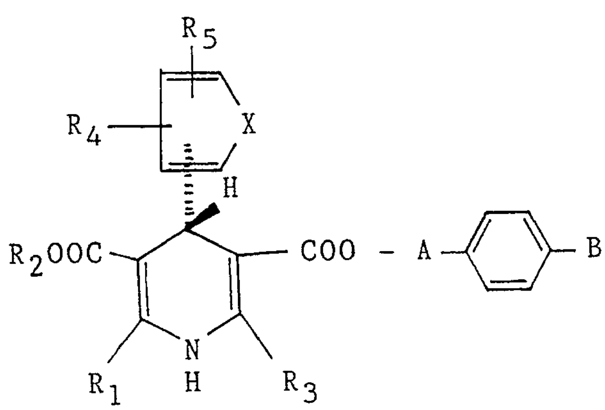

wherein $R_1$, $R_2$ and $R_3$ are the same or different, and each is alkyl, cycloalkyl or alkoxyalkyl; $R_4$ and $R_5$ are the same or different, and each is hydrogen atom, halogen, nitro, halogenated alkyl, alkylsulfonyl, halogenated alkoxy, alkylsulfinyl, alkyl, cycloalkyl, alkoxy, cyano, alkoxycarbonyl or alkylthio (wherein both of $R_4$ and $R_5$ are not hydrogen atoms at the same time); X is a group represented by vinylene or azomethine; A is alkylene; B is $-N(R_6)_2$ or

EP 0 424 901 A1

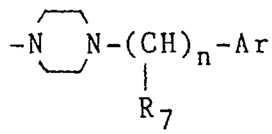

(wherein $R_6$ and $R_7$ are independently hydrogen atom, alkyl, cycloalkyl, aralkyl, aryl or pyridyl, Ar is aryl or pyridyl, and n is an integer of 0 to 2), acid addition salts thereof and pharmaceutical compositions containing same are disclosed. They exhibit potent and long-lasting antihypertensive action and can be used as a medicine for prevention and treatment of hypertension, and so on.

# DIHYDROPYRIDINE DERIVATIVES AND PHARMACEUTICAL COMPOSITIONS

## BACKGROUND OF THE INVENTION

The present invention relates to dihydropyridine derivatives, acid addition salts thereof and pharmaceutical compositions comprising said compounds as an active ingredient, which are novel and useful as pharmaceuticals.

As the compounds similar to dihydropyridine derivatives of the present invention, there have been known, for example, nifedipine, nicardipine, etc. While these compounds have been known to be useful as an antihypertensive agent, a peripheral and cerebral vasodilating agent and a coronary artery-treating (angina pectoris-treating) agent, dihydropyridine derivatives having still more excellent effect are demanded.

From such viewpoints, the present inventors have proposed dihydropyridine derivatives of the formula (II)

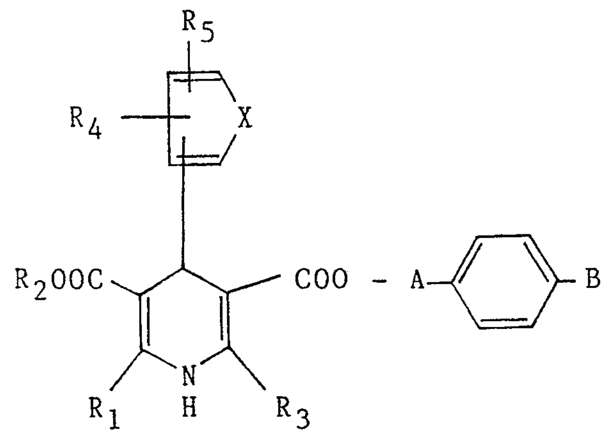

(II)

wherein $R_1$, $R_2$ and $R_3$ are the same or different, and each is alkyl, cycloalkyl or alkoxyalkyl; $R_4$ and $R_5$ are the same or different, and each is hydrogen atom halogen, nitro, halogenated alkyl, alkylsulfonyl, halogenated alkoxy, alkylsulfinyl, alkyl, cycloalkyl, alkoxy, cyano, alkoxycarbonyl or alkylthio (wherein both of $R_4$ and $R_5$ are not hydrogen atoms at the same time); X is a group represented by vinylene or azomethine; A is alkylene; B is $-N(R_6)_2$ or

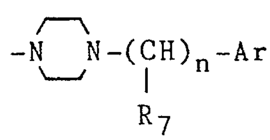

(wherein $R_6$ and $R_7$ are independently hydrogen atom, alkyl, cycloalkyl, aralkyl, aryl or pyridyl, Ar is aryl or pyridyl, and n is an integer of 0 to 2) and nontoxic acid addition salts thereof, which have an excellent calcium blocking action (Ca-antagonist), an antihypertensive action, a platelet aggregation-inhibiting action, a phosphodiesterase-inhibiting action and the like, and thus are useful as a medicine such as a coronary vasodilator, a cerebral hyperkinemic, antihypertensive, thrombosis-preventing or -treating agents, phosphodiesterase-inhibitor or the like (Japanese Patent Application Unexamined Publication No. 225356/1988).

The dihydropyridine derivatives (II) have a unique structure as compared with dihydropyridine compounds which have been so far concretely known, and have a specific activity due to such unique structure. Namely, the dihydropyridine derivatives (II) and their acid addition salts are specifically characterized in that they show a high organ and tissue-selectivity in vasodilating activities in particular and that they are highly safe due to their very low accute toxicity.

## SUMMARY OF THE INVENTION

The dihydropyridine derivatives (II) are, as discussed above, compounds having excellent characteristic properties. An object of the present invention is to provide dihydropyridine derivatives having still more excellent properties. Another object of the present invention is to provide pharmaceutical compositions comprising as an active ingredient said novel dihydropyridine derivatives.

The present inventors have conducted intensive studies in order to achieve the above-mentioned object and found that the dihydropyridine derivatives (II) possess optically active compounds and further that among such compounds, the compounds of formula (I) below possess extremely high activity, whereas the compounds of formula (I′) below scarcely exhibit activity, and developed the invention to completion.

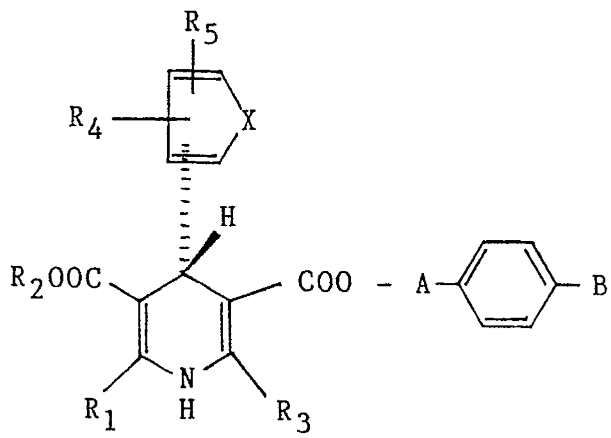

(I)

wherein $R_1$, $R_2$, $R_3$, $R_4$, $R_5$, X, A and B are as defined below.

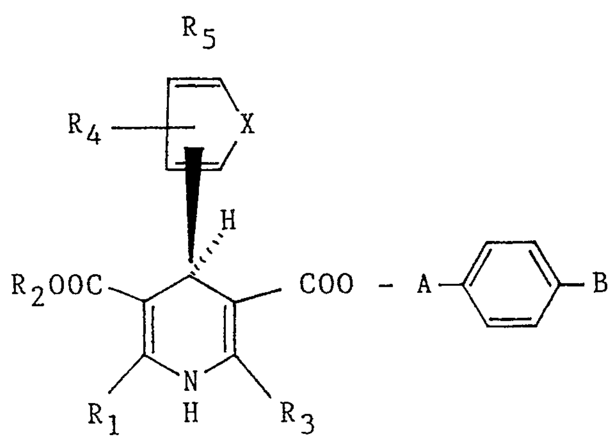

(I')

wherein $R_1$, $R_2$, $R_3$, $R_4$, $R_5$, X, A and B are as defined below.

## DETAILED DESCRIPTION OF THE INVENTION

The present invention relates to the dihydropyridine derivatives [hereinafter referred to as dihydropyridine derivatives (I)] of the formula

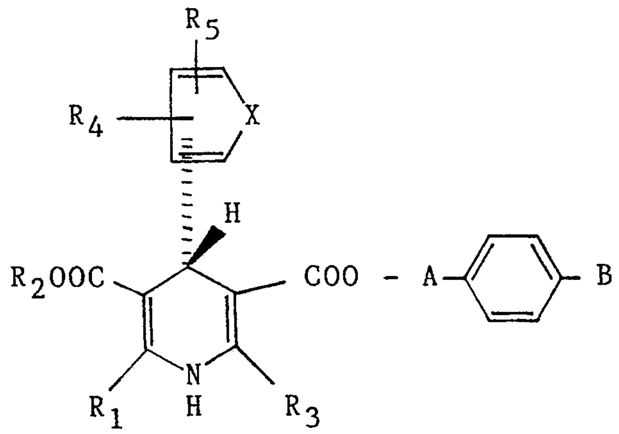

(I)

wherein $R_1$, $R_2$ and $R_3$ are the same or different, and each is alkyl, cycloalkyl or alkoxyalkyl; $R_4$ and $R_5$ are the same or different, and each is hydrogen atom, halogen, nitro, halogenated alkyl, alkylsulfonyl, halogenated alkoxy, alkylsulfinyl, alkyl, cycloalkyl, alkoxy, cyano, alkoxycarbonyl or alkylthio (wherein both of $R_4$ and $R_5$ are not hydrogen atoms at the same time); X is a group represented by vinylene or azomethine; A is alkylene; B is $-N(R_6)_2$ or

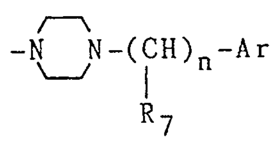

(wherein $R_6$ and $R_7$ are independently hydrogen atom, alkyl, cycloalkyl, aralkyl, aryl or pyridyl, Ar is aryl or pyridyl, and n is an integer of 0 to 2) and acid addition salts thereof. The present invention also relates to pharmaceutical compositions comprising as an active ingredient said compounds.

In the above formula, the alkyl represented by $R_1$, $R_2$ and $R_3$ may be straight or branched and particularly a lower alkyl ($C_{1-6}$), such as methyl, ethyl, propyl, isopropyl, butyl, isobutyl, sec-butyl, t-butyl, pentyl, isopentyl, neopentyl, hexyl, etc. is preferable, with further preference given to $C_{1-4}$ alkyls. The alkyl may have at the terminus a lower cycloalkyl ($C_{3-6}$) (e.g. cyclopropylmethyl, cyclobutylethyl, cyclopentylmethyl). As the cycloalkyl, preferred is a lower ($C_{3-6}$) cycloalkyl, such as cyclopropyl, cyclobutyl, cyclopentyl and cyclohexyl. As the alkoxyalkyl, preferred is an alkoxyalkyl having 3 to 7 carbon atoms in total, such as methoxyethyl, ethoxyethyl, propoxyethyl, isopropoxyethyl, butoxyethyl, methoxypropyl, 2-methoxy-1-methylethyl, 2-ethoxy-1-methylethyl, etc.

The substituents represented by $R_4$ and $R_5$ may be the same or different, and may be attached to the ring at any position. Preferably, they are attached to the ring at the 2- and 3-positions to the binding site with the dihydropyridine ring, and when one of $R_4$ or $R_5$ is hydrogen and the other is a substituent, it is attached at the 2- or 3-position. The halogens represented by $R_4$ and $R_5$ include fluorine atom, chlorine atom, bromine atom and iodine atom, of which fluorine and chlorine atoms are particularly preferable. As the alkyl and cycloalkyl, preferred are those mentioned above as the examples for $R_1$ to $R_3$. As the alkoxy and alkylthio, preferred are those having a lower alkyl ($C_{1-3}$), which are exemplified by methoxy, ethoxy, propoxy and isopropoxy, and methylthio, ethylthio, propylthio and isopropylthio, respectively. As the alkoxycarbonyl, there can be mentioned those having 2 to 4 carbon atoms such as methoxycarbonyl and ethoxycarbonyl. As the halogens of the halogenides, halogens mentioned above are exemplified. The halogenated alkyls include those in which a part of the hydrogen atoms are halogenated [e.g. $(CF_3)_2CHCH_2$-, $CF_3CH_2$-] and those in which all the hydrogen atoms are halogenated (e.g. trifluoromethyl). The halogenated alkoxys also include those in which a part of the hydrogen atoms are halogenated and those in which all the hydrogen atoms are halogentaed. As the alkoxy in the halogenated alkoxy, preferred are those having 1 to 3 carbon atoms. As the alkyl in the alkylsulfonyl, the alkylsulfinyl and the halogenated alkyl, there may be mentioned those shown above as the examples for $R_1$ to $R_3$.

As $R_4$ and $R_5$, particularly preferred are cyano and halogenated alkyls (particularly, trifluoromethyl).

As the alkyl and cycloalkyl represented by $R_6$ and $R_7$, there can be mentioned those shown above as the examples for $R_1$ to $R_3$. As the aralkyl, there can be mentioned phenyl $C_{1-3}$-alkyl such as benzyl, α-phenylethyl, β-phenylethyl and γ-phenylpropyl, and as the aryl, phenyl and naphthyl can be mentioned.

The aromatic ring of them may have the same or different substituents at an optional position of the ring. As the substituents on the aromatic ring, there may be mentioned, for example, those shown above as the examples for $R_4$ and $R_5$. The pyridyl includes 2-pyridyl, 3-pyridyl and 4-pyridyl, which may be substituted by the groups mentioned above as the examples for $R_4$ and $R_5$.

As the alkylene represented by A, preferable are those having 2 to 4 carbon atoms and they may be straight or branched. They are exemplified by ethylene, trimethylene, tetramethylene, 1,2-dimethylethylene, etc.

The aryl and pyridyl represented by Ar include, for example, those mentioned above as the examples for $R_6$ and $R_7$, and they may have the same substituents.

The ring represented by the formula

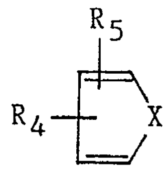

as the substituent at the 4-position of dihydropyridine means a benzene ring in case where X is vinylene (-CH=CH-), and a pyridine in case where X is azomethine (-CH=N-), and the ring may bind to the 4-position of the dihydropyridine at the optional position.

The substituents represented by $R_4$ and $R_5$ may be attached to the ring at any of the ortho-, metha- and para-positions relative to the carbon atom binding to the 4-position of the dihydropyridine, and are attached preferably to the ortho- and metha-positions of the ring, and when one of $R_4$ or $R_5$ is hydrogen and the other is a substituent, it is attached at the 2- or 3-position.

The dihydropyridine derivatives (I) can be produced, for example, by the following method.

Method 1

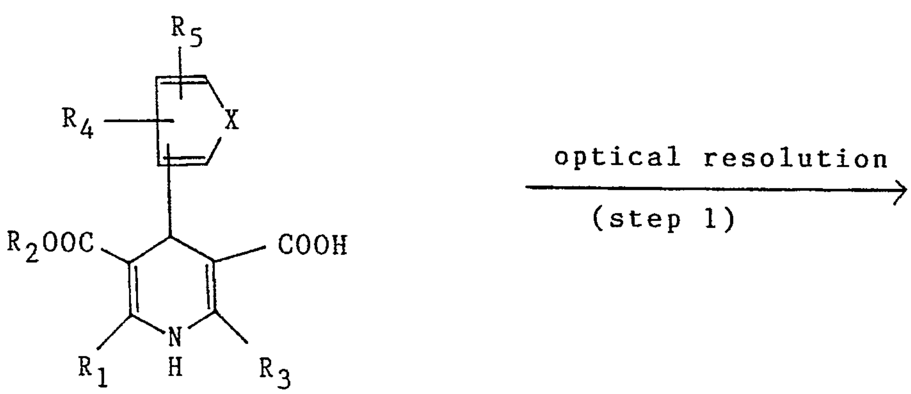


(1-1)

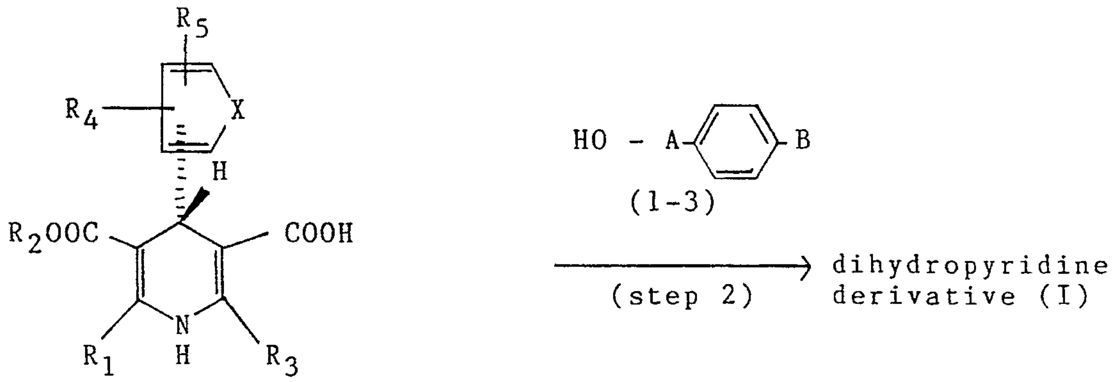


(1-2)

wherein $R_1$, $R_2$, $R_3$, $R_4$, $R_5$, X, A and B are as defined above.

Step 1 is directed to an optical resolution. More specifically, it is a step for forming a salt with an optically active amine to obtain compound (1-2).

As said amine, mention may be made of quinidine, cinchonidine, etc. Formation of salt with said amine and the optical resolution can be conducted according to a method known per se. The method described in Japanese Patent Application Unexamined Publication No. 185960/1986 may be exemplified.

Step 2 is conducted according to a known esterification reaction (Japanese Patent Application Unexamined Publication No. 260064/1986). Namely, a reactive derivative of carboxyl group of compound (1-2) (e.g. acid halide, acid anhydride) is reacted with compound (1-3) to produce dihydropyridine derivative (I). In this reaction, an acid may be used as a catalyst, and a condensing agent may also be used.

The dihydropyridine derivative (I) is produced as follows:

Compound (1-2), after being reacted with halogenated thionyl, etc. into an acid halide, is reacted with compound (1-3). The reaction between compounds (1-2) and (1-3) proceeds normally at a temperature from about 0° C to about 30° C, preferably, about 0° C to 20° C. As the solvent, any solvent can be used so long as it is inert to the reaction. Suitable solvents include, for example, ethers such as ethyl ether, dioxane, tetrahydrofuran, ethylene glycol monomethyl ether, ethylene glycol dimethyl ether, pyridine, N,N-dimethylformamide, dimethylsulfoxide, acetonitrile, chloroform, dichloromethane, etc. As for the amount of compounds (1-2) and (1-3), 1 to 1.5 moles of compound (1-3) is used based on 1 mole of compound (1-2). The reaction usually completes in about 1 to 30 hours.

<u>Method 2</u>

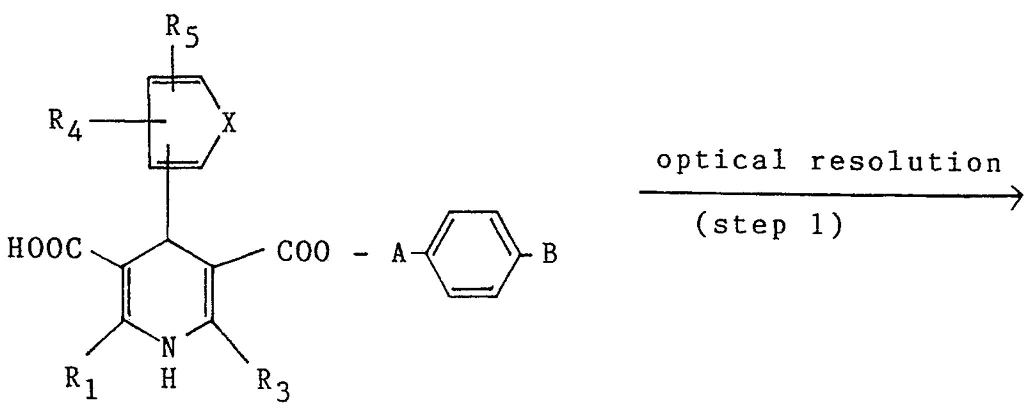


(2-1)

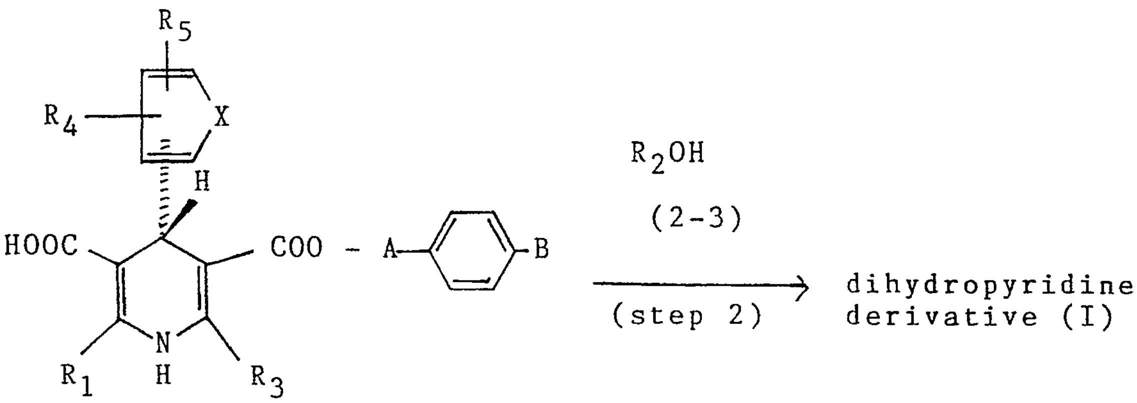


(2-2)

wherein $R_1$, $R_2$, $R_3$, $R_4$, $R_5$, X, A and B are as defined above.

The procedures are almost the same as in Method 1.

Method 3

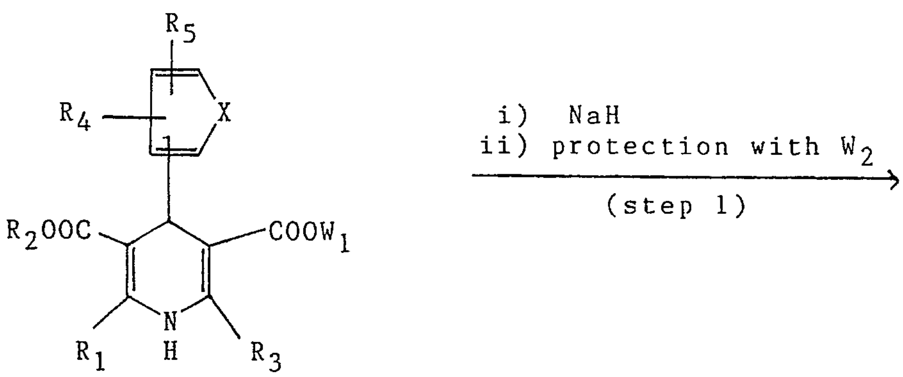


(3-1)

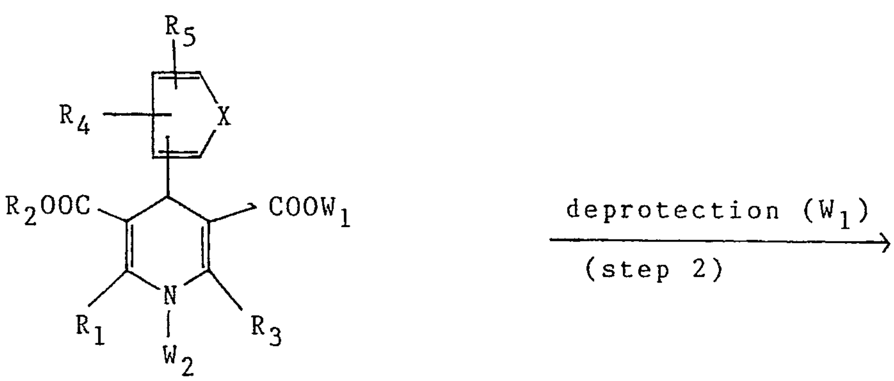


(3-2)

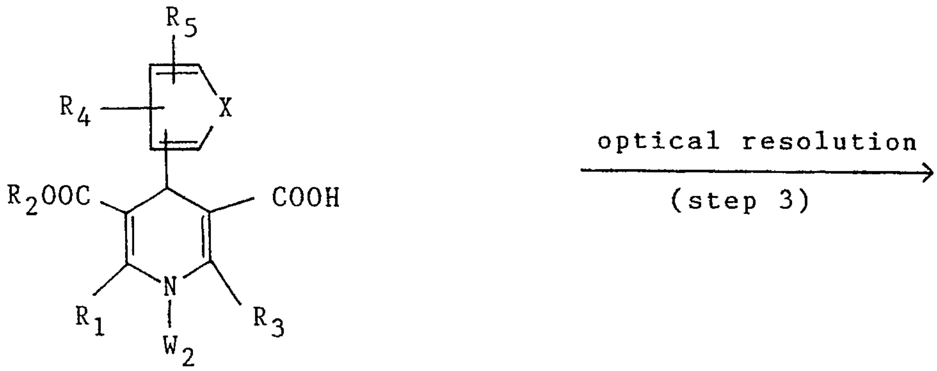


(3-3)

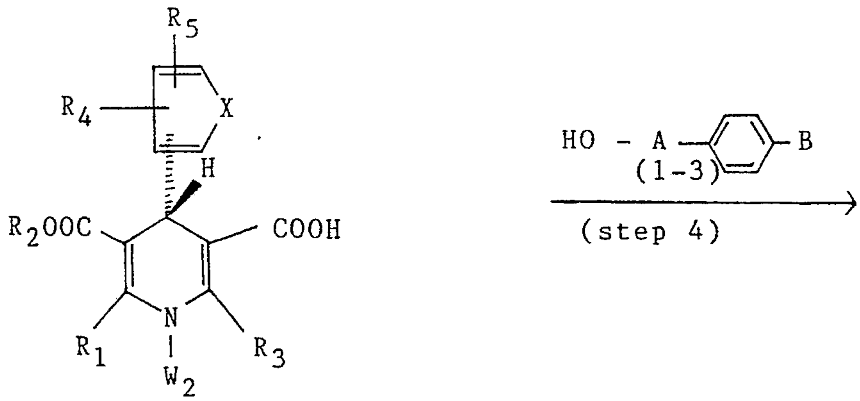


(3-4)

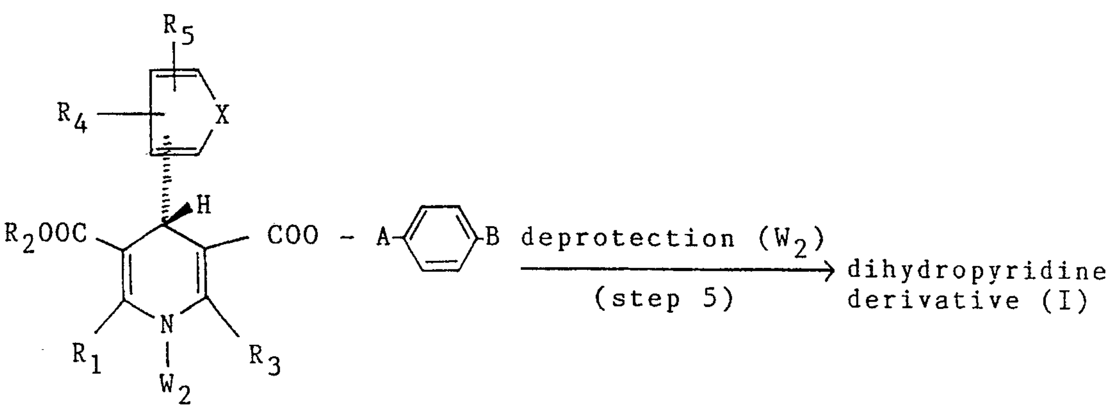


(3-5)

wherein $R_1$, $R_2$, $R_3$, $R_4$, $R_5$, X, A and B are as defined above, $W_1$ is a protective group for -COOH and $W_2$ is a protective group for >NH.

As regards $W_1$, no limitation is imposed so long as it is a protective group for -COOH and easily hydrolyzed under conditions wherein >N-$W_2$ is not hydrolyzed, such as alkaline conditions. Examples include those represented by the formulae

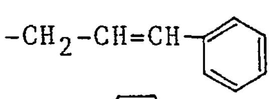, 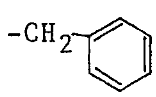, 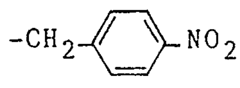, 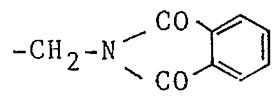,

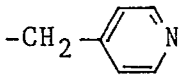,

$-CH_2CH_2CN$, $-CH_2CH_2N^{+}(CH_3)_3I^{-}$, $-CH_2CH_2S^{+}(CH_3)_2I^{-}$, $-CH_2CH_2OCOCH_3$, $-CH_2CH_2Si(CH_3)_3$.

As regards $W_2$, no limitation is imposed so long as it is a protective group for >NH and hydrolyzed under acidic conditions. Examples include those represented by the formulae $-CH_2OCH_3$, $-CH_2OCH_2CH_3$, $-CH_2N(CH_3)_2$,

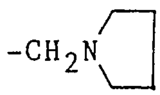 ,

$-CH_2COOH$, $-CH_2OCOCH_3$, $-CH_2OCOC(CH_3)_3$.

Compound (3-1) can be prepared by a known method. For example, USPs 4849429, 4910195 and 4886819 disclose such method.

Step 1 is a process for protecting the 1-position secondary amino group (>NH) with a protective group ($W_2$) and is conducted in accordance with the method described in Chem. Pharm. Bull., 27(6) , 1426-1440 (1979), etc. That is, compound (3-1) is reacted with NaH. In this reaction, NaH is used in about 1.3 to 1.8 equivalent amount, and an organic solvent such as tetrahydrofuran (THF) is preferably used. The reaction tem perature is near room temperature and the reaction time is about 10 minutes.

In protecting with $W_2$, a reagent is used in about 2.2 equivalent amount, the reaction temperature ranges from -40 $^{\circ}$C to room temperature and the reaction time is from 30 minutes to 2 hours.

Step 2 is a process for eliminating the protective group for -COOH, which is usually conducted by alkali hydrolysis. That is, it is a reaction with an aqueous solution of sodium hydroxide in an equivalent to 20 equivalent amount in an organic solvent such as dioxane, ethanol, THF, etc. at a temperature between room temperature and 50 $^{\circ}$C.

Thereafter, the optical resolution is conducted in step 3 in the same manner as in Method 1.

The reaction between compounds (3-4) and (1-3) in step 4 is also conducted in the same manner as in Method 1. The acid hydrolysis is conducted by a known method in the final step.

Step 5 is a process for eliminating the protective group for >NH, which is normally conducted by acid hydrolysis, that is, the reaction with about 1N hydrochloric acid in an organic solvent such as acetone, etc. at a temperature between 0 $^{\circ}$C and room temperature.

## Method 4

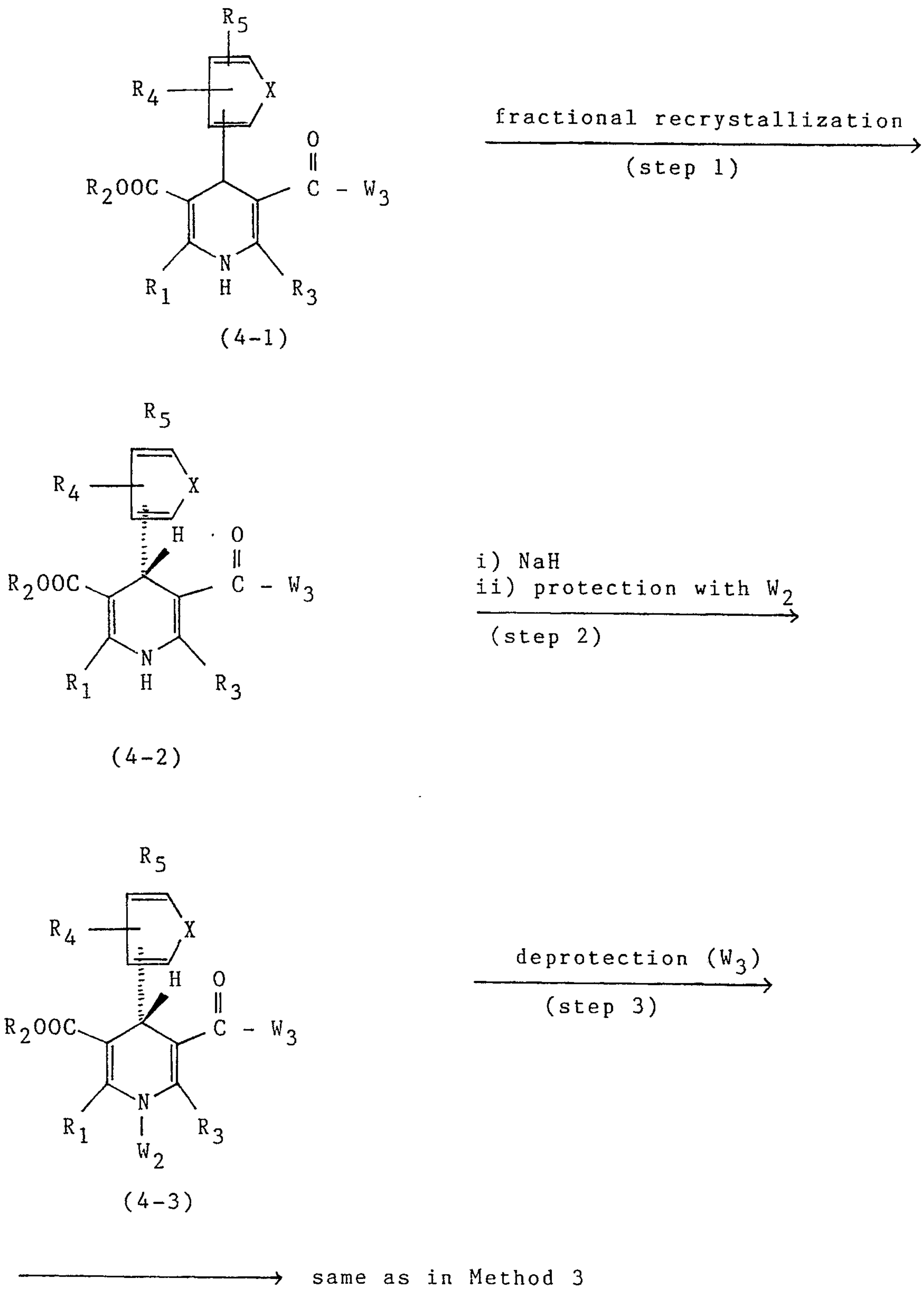


(4-1)

(4-2)

(4-3)

wherein $R_1$, $R_2$, $R_3$, $R_4$, $R_5$, X and $W_2$ are as defined above and $W_3$ is an optically active alcohol residue.

No restriction is imposed on $W_3$ so long as compound (4-1) is converted to a mixture of diastereomers by the introduction thereof, and $W_3$ is a group which is easily hydrolyzed under alkaline conditions, thereby regenerating the carboxylic acid. Examples thereof include (R)-(-)- and (5)-(+)-1-methoxycarbonylpropyl-2-oxy, (R)-(-)- and (S)-(+)-2-methoxycarbonylpropoxy, etc.

Compound (4-1) can be prepared by a known method, for example, the methods described in USPs 4849429, 4910195 and 4886819.

Step 1 is for separating a mixture of diastereomers by fractionation recrystallization.

Protection of >NH with $W_2$ in step 2 is conducted according to the method in Method 3.

Thereafter, deprotection of $W_3$ is conducted in the same manner as in the deprotection of $W_1$ by alkali hydrolysis, etc. The same procedure follows hereafter as in Method 3.

The dihydropyridine derivatives (I) thus produced can be purified to a desired extent by a known method such as concentration, extraction, chromatography, reprecipitation, recrystallization, and so on.

Besides, since the dihydropyridine derivatives (I) have basic groups, they can be converted into acid addition salts thereof by a known means. As regards such salts, there is no limitation imposed thereon so long as they are pharmacologically acceptable nontoxic salts, which are exemplified by salts with inorganic acids (e.g. hydrochloride, hydrobromide, phosphate, sulfate) and those with organic acids (e.g. acetate, succinate, maleate, fumarate, malate, tartrate).

The dihydropyridine derivatives (I) and acid addition salts thereof of the present invention are extremely low in toxicity and have an antihypertensive action, a peripheral vasodilating action, a coronary artery-dilating action, a cerebral vasodilating action and other actions which are potent and lasting in mammals (e.g. mouse, rat, rabbit, dog, cat, human). Thus, they are useful as a medicine for prophylaxis and treatment of hypertension, ischemic cardiac diseases (angina pectoris, myocardial infarction, etc.), cerebral and peripheral circulation disturbances (cerebral infarction, temporary cerebral ischemic spasm, etc.) and sequelae caused by cerebral circulation disturbances (obnubilation, respiratory disorder, paresthesia, lalopathy, paralysis, paropsis, decrease of subjective symptoms and hyponea).

In addition, the dihydropyridine derivatives (I) and acid addition salts thereof are useful for prevention and treatment of vascular contraction, for example, cerebrovascular contraction (e.g. cerebrovascular contraction caused by cerebral hemmorrhage, subarachnoid hemorrhage, etc.), coronary vascular contraction [stroke caused by contraction on deoppilation of stegnosis site by laser cautery, etc., specifically the one which is intractable and to which nitroglycerin is inef fective, including postoperative coronary contraction, angina pectoris induced by coronary contraction (coronary pectoris vasomotoria)] and so on.

In particular, the dihydropyridine derivatives (I) and acid addition salts thereof are more excellent in their potency and duration of pharmacological actions, particularly Ca-antagonistic action, as compared with previously known dihydropyridine derivatives (e.g. nifedipine, nicardipine). Thus, they exhibit a stable antihypertensive action by a few dosages (once or twice a day) when, for example, used as a medicine for the prophylaxis or treatment of hypertension.

When the dihydropyridine derivatives (I) and their acid addition salts are used as medicines mentioned above, they can be mixed with pharmaceutically required ingredients such as pharmacologically acceptable additives (e.g. carrier, excipient, diluent) to give a pharmaceutical composition in a form such as powders, granules, tablets, capsules or injection, which can be orally or parenterally administered. The dihydropyridine derivatives (I) and their acid addition salts are incorporated in the above-mentioned pharmaceutical compositions in a pharmaceutically effective amount. While the dosage varies depending on the administration route, gravity of the diseases, body weight or age of patients and the like, they can be orally administered to an adult suffering from hypertension in an amount of 0.05 to 20 mg/kg body weight/day, preferably 0.1 to 4 mg/kg body weight/day in one to several divided doses a day.

The present invention is hereinbelow detailedly explained by illustrating working examples and experiment example. However, the present invention is not limited thereto.

## Example 1

To 1,4-dihydro-3-methoxycarbonyl-2,6-dimethyl-4-(3-nitrophenyl)-5-pyridinecarboxylic acid [hereinafter referred to as compound (1)] (20.84 g, 62.6 mmol) and quinidine (20.309 g, 62.6 mmol) was added dimethylformamide (DMF) (37 ml) and the mixture was heated to about 90 °C for dissolution. To the reaction mixture was added water (25 ml) little by little, and kept standing at room temperature for 20 hours. The precipitated clay substance was stirred at room temperature for 2 hours, after which it was suction-filtered, washed with water, dissolved in acetone (300 ml) and subjected to distilling off under reduced pressure. The residue was recrystallized from DMF (60 ml)-water (about 50 ml) to give 14.903 g of quinidine salt of (-)-rich compound (1). The obtained crystals (14.637 g) was recrystallized from DMF (42 ml)-water (28 ml), and the thus-obtained crystals (10.677 g) was again recrystallized from DMF (31 ml)-water (21 ml) to give 9.423 g of pure quinidine salt of (-)-compound (1) as yellow slightly needle-like crystals. To the quinidine salt of (-)-compound (1) (9.172 g) was added water (31 ml) containing sodium hydroxide (35% aqueous solution, 1.4 ml) and the mixture was washed with dichloromethane. The water

layer was acidified with concentrated hydrochloric acid (1.8 ml) and stirred for 70 minutes under ice-cooling. The precipitated crystals were suction-filtered, washed with water and dried to give 3.957 g (38%) of (R)-(-)-compound (1), m.p. 168 - 169.5 °C.

$[\alpha]_D^{20}$ -25.9° (C 0.50, acetone)

IR and $^1$H-NMR were the same as for the racemate.

The mother liquor from the first recrystallization mentioned above was distilled off under reduced pressure. To the residue was added water (200 ml) containing sodium hydroxide (35% aqueous solution, 11 ml) and the mixture was washed with dichloromethane. The water layer was acidified with concentrated hydrochloric acid (11 ml) and stirred for 40 minutes under ice-cooling. The precipitated crystals were suction-filtered and dried to give 8.276 g of (+)-rich compound (1). To the obtained (+)-rich compound (1) (8.024 g, 24.1 mmol) and cinchonidine (7.108 g, 24.1 mmol) was added DMF (23 ml) and the mixture was heated to 90 - 100 °C for dissolution. To the reaction mixture was added water (15 ml) at the same temperature and kept standing at room temperature for 20 hours. The precipitated crystals were suction-filtered, washed with DMF and water (3 : 2), and dried to give 10.676 g of cynchonidine salt of (+)-rich compound (1). The obtained crystals (10.426 g) were recrystallized from DMF (30 ml)-water (20 ml) to give 8.817 g of pure cinchonidine salt of (+)-compound (1) as pale yellow slightly needle-like crystals. To the obtained cynchonidine salt (8.567 g) of (+)-compound (1) was added water (29 ml) containing sodium hydroxide (35% aqueous solution, 1.3 ml) and the mixture was washed with dichloromethane. The water layer was acidified with concentrated hydrochloric acid (1.6 ml) and stirred under ice-cooling for 70 minutes. The precipitated crystals were suction-filtered, washed with water and dried to give 3.546 g (34%) of (S)-(+)-compound (1) as slightly yellow powder, m.p. 170 - 171 °C.

$[\alpha]_D^{20}$ +26.1° (C 0.50, acetone)

IR and $^1$H-NMR were the same as for the racemate.

<u>(R)-(-)-4-(4-benzhydrylpiperadino)phenetyl methyl 1,4-dihydro-2,6-dimethyl-4-(3-nitrophenyl)-3,5-pyridinedicarboxylate hydrochloride [(R)-(-)-compound (3)]</u>

(S)-(+)-Compound (1) (1.513 g, 4.55 mmol) was suspended in dichloromethane (8.3 ml) and DMF (2.1 ml) in a nitrogen atmosphere. The reaction mixture was ice-cooled, and thereto was dropwise added thionyl chloride (563 mg, 0.34 ml, 4.74 mmol) at 3-5 °C. After addition, the mixture was stirred at said temperature for 1 hour. To the mixture was dropwise added 4-(4-benzhydrylpiperadino)phenethyl alcohol [hereinafter referred to as compound (2)] (1.696 g, 4.55 mmol) in dichloromethane (5.2 ml) at said temperature. After addi tion, the mixture was stirred at the same temperature for 1 hour and kept standing for 16 hours. To the mixture was added dichloromethane (30 ml). The mixture was washed with water, dried and distilled off under reduced pressure. To the residue was added ethyl acetate (45 ml), and the mixture was washed with 5% potassium carbonate solution and saturated brine, after which it was dried and distilled off under reduced pressure. The obtained residue (3.170 g) was separated by column chromatography (silica gel : ethyl acetate-n-hexane (2 : 3)], and the obtained (R)-(-)-compound (3) (free) was further purified by HPLC to give 2.217 g (71%) of pure (R)-(-)-compound (3) (free). To the (R)-(-)-compound (3) (free) (696 mg, 1.01 mmol) was added dichloromethane (5 ml). After dissolution, thereto was added a 1,2-dimethoxyethane solution of hydrogen chloride (2.53 N, 0.84 ml) at room temperature. The mixture was stirred at room temperature for 7 minutes, at a temperature between 40 and 35 °C for 20 minutes and at a temperature between 34 °C and room temperature for 3 hours. The solvent was distilled off under reduced pressure, and the obtained residue was dried to give 690 mg (71%) of (R)-(-)-compound (3).

IR($CHCl_3$) : 2400, 1690, 1525, 1350 $cm^{-1}$

$^1$H-NMR$\delta$ : 2.26, 2.36 (each 3H, s), 2.82 (2H, t, J=6.5Hz), 2.95-3.25 (2H), 3.4-3.6 (4H), 3.64 (3H, s), 3.8-4.1 (2H), 4.24 (2H, t, J=6.5Hz), 4.87 (1H, d, J=8.5Hz), 5.06 (1H, s), 6.33 (1H, s), 6.80, 7.01 (4H, $A_2B_2$, q, J=8Hz), 7.25-7.6 (8H, m), 7.8-8.1 (6H, m), 13.18 (1H, brs).

$[\alpha]_D^{20}$ -29.9° (C 0.50, acetone)

<u>(S)-(+)-4-(4-benzhydrylpiperadino)phenetyl methyl 1,4-dihydro-2,6-dimethyl-4-(3-nitrophenyl)-3,5-pyridinedicarboxylate hydrochloride [(S)-(+)-compound (3)]</u>

The same procedure was repeated as in the case with (S)-(+)-compound (1), using (R)-(-)-compound (1) (1.507 g, 4.53 mmol) to give 677 mg (71%) of (S)-(+)-compound (3) as yellow powder.

$[\alpha]_D^{20}$ +29.7° (C 0.50, acetone)

IR and $^1$H-NMR were the same as for (R)-(-)-compound 3. The optical purity was not less than 98% for both.

## Experiment Example

The subject rats anesthetized with pentobarbital-Na (50 mg/kg, i.p.) were fixed at the dorsal position, and the blood pressure was successively recorded on a polygraph via polyethylene catheter inserted in left common carotid artery and piezoelectric transducer. The drug was bolus- administered at a dose of 1 ml/kg through a wing needle dwelled in tail vein.

As the subject animal, male Wistar rats (about 300 g body weight, 4 rats per group) were used.

The test drug was prepared by dissolving 10 mg thereof in 0.3 ml of ethanol and 0.1 ml of Tween 80 and diluting with saline. The results are summarized in Table 1.

Table 1

(mean ± SE ; n = 4)

| Drug | Dose (μg/kg) | SBP | | DBP | |
|---|---|---|---|---|---|
| | | % change | ED30 | % change | ED30 |
| (S)-(+)-compound 3 | 10<br>30<br>100 | 86±3<br>86±3<br>71±5 | 160 | 89±1<br>74±2<br>55±4 | 37 |
| (R)-(-)-compound 3 | 100<br>1000 | 93±2<br>93±3 | - | 94±1<br>91±2 | - |
| compound 3 | 30<br>100<br>300 | 79±1<br>76±4<br>72±3 | 630 | 75±5<br>61±2<br>53±3 | 46 |

In the table, % change is the blood pressure expressed in % relative to that before administration of the drug which was taken as 100%, $ED_{30}$ is the estimated dose amount (μg/kg body weight) necessary for 30% reduction of blood pressure, SBP means systolic blood pressure and DBP means diastolic blood pressure.

## Formulation Example

| Tablets | | |
|---|---|---|
| (1) | (S)-(+)-compound (3) | 10 g |
| (2) | Fine particles No. 209 for direct compression (Manufactured by Fuji Chemical Corp.)<br>Magnesium metasilicate aluminate 20%<br>Corn starch 30%<br>Lactose 50% | 110 g |
| (3) | Crystalline cellulose | 60 g |
| (4) | CMC calcium | 18 g |
| (5) | Magnesium stearate | 2 g |

(1), (3) and (4) were passed through a sieve of 100 mesh in advance. After (1), (3), (4) and (2) were dried, whereby the water-content was reduced to the specific degree, they were admixed in the above-mentioned weight proportions with the use of a mixer. (5) was added to the powders mixed wholly homogeneously; and the mixture was mixed for a short time (30 seconds). The mixed powders were compressed into tablets weighing 200 mg per tablet.

These tablets may be coated with enteric film-coating agents (e.g. polyvinylacetaldiethylaminoacetate) and edible coloring agents in conventional use.

| Capsules | | |
|---|---|---|
| (1) | (S)-(+)-compound (3) | 50 g |
| (2) | Lactose | 950 g |
| (3) | Magnesium stearate | 20 g |

The above ingredients were weighed and mixed homogeneously and the mixed powders were filled in hard gelatin capsules in an amount of 200 mg per capsule.

| Injections | | |
|---|---|---|
| (1) | (S)-(+)-compound (3) | 5 mg |
| (2) | Glucose | 100 mg |
| (3) | Physiological saline | 10 ml |

The mixture of the above-mentioned ingredients was filtered with a membrane filter and again subjected to sterile filtration. The filtrate was poured, under sterile conditions, into vials, which were filled with nitrogen gas. Thereafter, the vials were sealed to give intravenous injections.

## Claims

(1) A dihydropyridine derivative of the formula

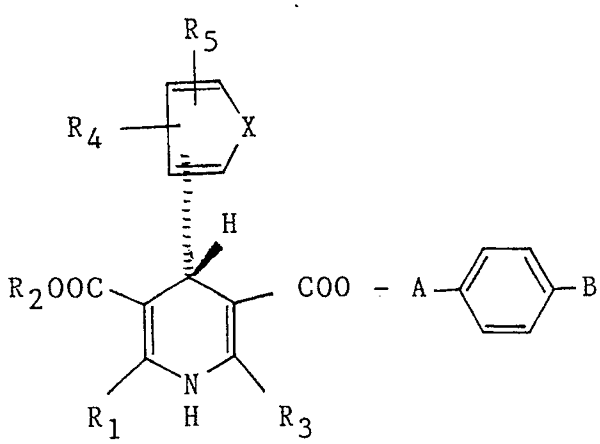

(I)

wherein $R_1$, $R_2$ and $R_3$ are the same or different, and each is alkyl, cycloalkyl or alkoxyalkyl; $R_4$ and $R_5$ are the same or different, and each is hydrogen atom, halogen, nitro, halogenated alkyl, alkylsulfonyl, halogenated alkoxy, alkylsulfinyl, alkyl, cycloalkyl, alkoxy, cyano, alkoxycarbonyl or alkylthio (wherein both of $R_4$ and $R_5$ are not hydrogen atoms at the same time); X is a group represented by vinylene or azomethine; A is alkylene; B is $-N(R_6)_2$ or

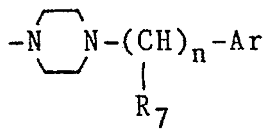

(wherein $R_6$ and $R_7$ are independently hydrogen atom, alkyl, cycloalkyl, aralkyl, aryl or pyridyl, Ar is aryl or pyridyl, and n is an integer of 0 to 2), or an acid addition salt thereof.

(2) A dihydropyridine derivative as claimed in Claim 1 wherein as regards $R_1$, $R_2$ and $R_3$ the alkyl has 1 to 6 carbon atoms and may optionally have $C_{3-6}$ lower cycloalkyl at the terminus, the cycloalkyl has 3 to 6 carbon atoms and the alkoxyalkyl has 3 to 7 carbon atoms, or an acid addition salt thereof.

(3) A dihydropyridine derivative as claimed in Claim 1, wherein the substituents represented by $R_4$ and $R_5$ are at the 2- and the 3-positions to the binding site with a dihydropyridine ring, and when one of $R_4$ or $R_5$ is hydrogen and the other is a substituent, said substituent is attached at the 2- or 3-position, or an acid addition salt thereof.

(4) A dihydropyridine derivative as claimed in Claim 1, wherein as regards $R_4$ and $R_5$, the alkyl in alkyl, alkylsulfonyl, alkylsulfinyl and halogenated alkyl has 1 to 6 carbon atoms and may optionally have $C_{3-6}$ lower cycloalkyl at the terminus, the cycloalkyl has 3 to 6 carbon atoms, the alkoxy in alkoxy and halogenated alkoxy has 1 to 3 carbon atoms, the alkylthio has 1 to 3 carbon atoms and the alkoxycarbonyl has 2 to 4 carbon atoms, or an acid addition salt thereof.

(5) A dihydropyridine derivative as claimed in Claim 1, wherein $R_4$ and $R_5$ are respectively cyano, halogenated alkyl or nitro, or an acid addition salt thereof.

(6) A dihydropyridine derivative as claimed in Claim 1, wherein as regards $R_6$ and $R_7$, the alkyl has 1 to 6 carbon atoms and may optionally have $C_{3-6}$ lower cycloalkyl at the terminus, the cycloalkyl has 3 to 6 carbon atoms, the aralkyl is phenyl- $C_{1-3}$-alkyl and the aryl is phenyl or naphthyl, or an acid addition salt thereof.

(7) A dihydropyridine derivative as claimed in Claim 1, wherein A is ethylene, or an acid addition salt thereof.

(8) A dihydropyridine derivative as claimed in Claim 1, wherein X is vinylene, or an acid addition salt thereof.

(9) A dihydropyridine derivative as claimed in Claim 1, wherein Ar is phenyl, or an acid addition salt thereof.

(10) A dihydropyridine derivative as claimed in Claim 1, wherein both $R_7$ and Ar are phenyl optionally having substituents and n is 1, or an acid addition salt thereof.

(11) A dihydropyridine derivative as claimed in Claim 1, wherein both $R_7$ and Ar are phenyl, X is vinylene, A is ethylene and n is 1, or an acid addition salt thereof.

(12) A pharmaceutical composition comprising dihydropyridine derivative as claimed in Claim 1 or its salt and pharmacologically acceptable additive.

(13) A pharmaceutical composition as claimed in Claim 12, which is a calcium-antagonist.

European Patent Office

# EUROPEAN SEARCH REPORT

Application Number

**EP 90 12 0381**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y | EP-A-0 257 616 (GREEN CROSS)<br>* Entire document * | 1-13 | C 07 D 211/90<br>C 07 D 401/12<br>A 61 K 31/445 |
| Y | EP-A-0 273 349 (SYNTEX)<br>* Example 2: "Resolution of enantiomers using cinchonidine" * | 1-13 | |
| Y | EP-A-0 296 316 (BYK-GULDEN)<br>* Entire document; especially page 13, line 31 - page 18, line 15; pages 18-25, examples * | 1-13 | |

TECHNICAL FIELDS SEARCHED (Int. Cl.5)

C 07 D 211/00
C 07 D 401/00

The present search report has been drawn up for all claims

| Place of search | Date of completion of search | Examiner |
|---|---|---|
| The Hague | 23 January 91 | KISSLER B.E. |

CATEGORY OF CITED DOCUMENTS

X: particularly relevant if taken alone
Y: particularly relevant if combined with another document of the same catagory
A: technological background
O: non-written disclosure
P: intermediate document
T: theory or principle underlying the invention

E: earlier patent document, but published on, or after the filing date
D: document cited in the application
L: document cited for other reasons

&: member of the same patent family, corresponding document